(19) Europäisches Patentamt
European Patent Office
Office européen des brevets

(11) Publication number: **0 061 737 A1**

(12) **EUROPEAN PATENT APPLICATION**

(21) Application number: **82102532.7**

(22) Date of filing: **25.03.82**

(51) Int. Cl.³: **C 12 P 19/56**
//C07H15/24, (C12P19/56, C12R1/465)

(30) Priority: **28.03.81 JP 45789/81**

(43) Date of publication of application: **06.10.82 Bulletin 82/40**

(84) Designated Contracting States: **DE FR GB IT**

(71) Applicant: **SANRAKU-OCEAN CO., LTD.**
**15-1, Kyobashi 1-chome Chuo-ku**
**Tokyo(JP)**

(72) Inventor: **Oki, Toshikazu**
**1356-59 Kamigo-machi Totsuka-ku**
**Yokohama-shi Kanagawa-ken(JP)**

(72) Inventor: **Takatsuki, Yukio**
**Sanraku-Ocean Co Ltd. Hodogayaryo 555 Nagata-machi**
**Minami-ku Yokohama-shi Kanagawa-ken(JP)**

(72) Inventor: **Yoshimoto, Akihiro**
**Shonan-Life-Town G-8-7, 673-2, Endo**
**Fujisawa-shi Kanagawa-ken(JP)**

(72) Inventor: **Ishikura, Tomoyuki**
**22-32, Kowada 1-chome Chigasaki-shi**
**Kanagawa-ken(JP)**

(72) Inventor: **Takeuchi, Tomio**
**5-1-11 Higashigotanda Shinagawa-ku**
**Tokyo(JP)**

(72) Inventor: **Umezawa, Hamao**
**23 Toyotamakita 4-chome Nerima-ku**
**Tokyo(JP)**

(74) Representative: **Müller-Boré, Deufel, Schön, Hertel**
**Patentanwälte**
**Postfach 86 07 20 Siebertstrasse 4**
**D-8000 München 86(DE)**

(54) **Process for production of adriamyin.**

(57) Disclosed is a process for production of adriamycin which consists of converting a compound presented by formula (I)

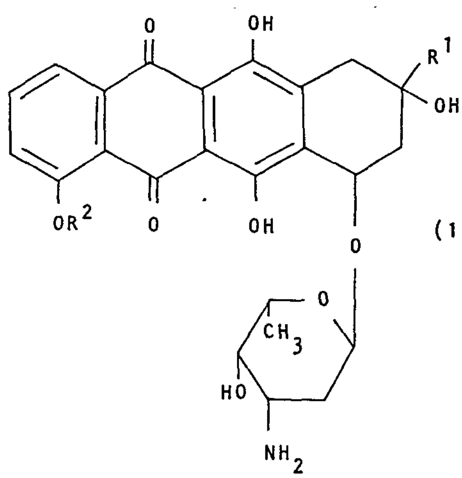

(1)

wherein

$R^1$ is acetyl, 1-hydroxyethyl or ethyl; and $R^2$ is a hydrogen atom or methyl; but, when $R^1$ is ethyl, $R^2$ is methyl,

to adriamycin with the culture of its processed matters of a microorganism having such conversion ability.

EP 0 061 737 A1

BACKGROUND OF THE INVENTION

(1) Field of the invention

The present invention relates to a novel process for production of adriamycin, a potent antitumor agent, which consists of biochemically converting other anthracycline compounds to adriamycin.

(2) Description of the prior art

A fermentative method for production of adriamycin in which Streptomyces peucetius subsp. caesius ATCC 27952 has been described in U.S.P. 3,590,028. A chemical process for preparation of adriamycin from daunomycin has been disclosed in U.S.P. 3,803,124.

SUMMARY OF THE INVENTION

The present invention relates to a novel process for production of adriamycin. More particularly, it relates to a process for production of adriamycin which consists of biochemically converting an anthracycline compound presented by formula (I)

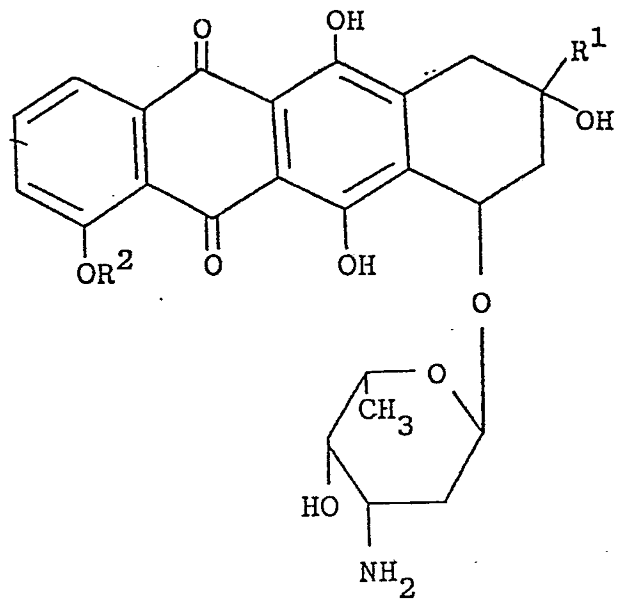

(I)

wherein

$R^1$ is acetyl, 1-hydroxyethyl or ethyl;

and $R^2$ is a hydrogen atom or methyl;

but, when $R^1$ is ethyl, $R^2$ is methyl,

to adriamycin presentd by formula (II)

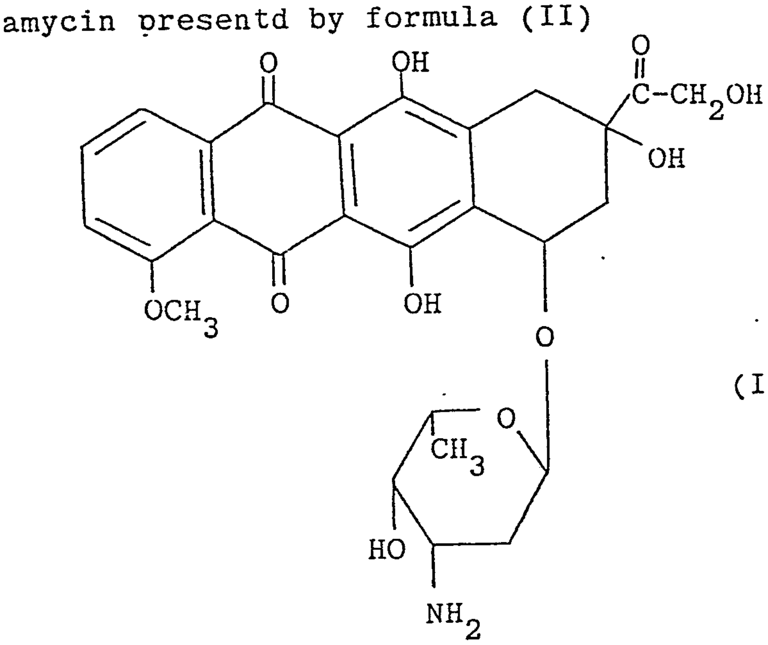

(II)

in the presence of the mycelium or its processed matters of a streptomycete having such conversion ability whereby

adriamycin is isolated and purified by conventional methods.

DETAILED DESCRPTION OF THE INVENTION

Adriamycin is an anthracycline antibiotic having a potent and broad antitumor activity and has widely been used as a valuable chemotherapeutic agent in the clinical treatment of tumors.

In the course of detailed studies on the biosynthesis of the anthracycline family of antibiotics by using various anthracycline-producing organisms and their mutants, the present authors have reported a biosynthetic route of daunomycin from aklavinone *via* $\varepsilon$-rhodomycinone and 13-deoxodaunomycin (for example, J. Antibiotics Vol. 33, 1158 & 1199 (1980)).

Based on these findings, the present authors have continued their studies to develop a more advantageous process for production of adriamycin. The present invention depends on a discovery that adriamycin can be produced more efficiently by a biochemical conversion method in which an anthracycline antibiotic presented by formula (I) (substrate) (typical examples of such compounds are listed in Table 1) is treated under non-reproductive conditions with the culture or its processed matters of a known adriamycin-producing organism or its mutants.

Table 1

| Formula I / Compounds | $R^1$ | $R^2$ | Reference |
|---|---|---|---|
| Daunomycin | $-COCH_3$ | $-CH_3$ | Listed above. |
| Dihydrodaunomcyin | $-CH(OH)-CH_3$ | $-CH_3$ | Jap. P. 48-2357 (1973) |
| Carminomycin | $-COCH_3$ | -H | J. Antibiotics 27, 254 (1974) |
| Dihydrocarmino-mycin | $-CH(OH)-CH_3$ | -H | Antibiotiki 21, 1008 (1976) |
| Deoxodaunomycin | $-CH_2CH_3$ | $-CH_3$ | J. Med. Chem. 21, 280 (1978) |

A wide range of microorganisms are employable in the present invention, as far as they have an ability to convert an anthracycline compound presented by formula (I) to adriamycin presented by formula (II) under non-reproductive conditions. It is more advantageous, however, to use adriamycin-producing streptomycetes or their mutant strains. Favorable examples of such streptomycetes are Streptomyces peucetius subsp. caesius ATCC 27952, Streptomyces peucetius subsp. carneus ATCC 21354 and their mutants that produce no anthracycline-type pigments.

Such mutant strains can be obtained by conventional

mutation methods such as treatment with mutagens (for example, nitrosoguanidine, diepoxyethane and nitrite) and irradiation of ultraviolet light or alpha-, gamma- or X-ray.

A method for isolation of useful mutants for the present invention is more concretely explained in the following:

A loopful of spores were collected from an agar slant culture of Streptomyces peucetius subsp. caesius ATCC 27592 and were inoculated into a test tube containing 5 ml of YS broth (0.3 % yeast extract, 1 % soluble starch; pH 7.2). After shaking cultivation for 2 days at 28°C on a reciprocal shaker, the culture was treated for 30 seconds in a sonicator (Tomy Seiko UR-200P), passed through a sterile glass tube (1.5 x 2.0 cm) containing cotton and then centrifuged for collection of the cells. The cells were suspended in 5 ml of 0.1 M Tris-HCl buffer, pH 8.5, containing 1 mg/ml N-methyl-N'-nitro-N-nitrosoguanidine and were kept shaken for one hour at 28°C. The precipitate recovered by centrifugation was suspended in 5 ml of physiological saline and, if necessary after appropriate dilution with the saline, spread on YS agar plates (YS broth + 1.5 % agar) for colony isolation. The agar plates were incubated at 28°C for 5 days. Each colony was inoculated on a YS agar slant and into a test tube containing 3 ml of YS

broth (described above). The test tubes were shake-cultured at 28°C for 2 days and the whole cultures were transferred into 250 ml flasks containing 30 ml of fermentation medium having the composition described below. The flasks were incubated at 28°C for 5 days on a rotary shaker.

Fermentation medium

| | |
|---|---|
| Sucrose | 5 % |
| Soybean meal (ESUSAN MEAT; Ajinomoto Co.) | 2 % |
| Corn steep liquor | 0.2 % |
| Yeast extract | 0.2 % |
| Dipotassium phosphate | 0.1 % |
| Magnesium sulfate | 0.05 % |
| Calcium carbonate | 0.2 % |
| Mineral solution* | 0.2 % |

pH 7.4

(* Mineral solution: 2.8 g $CuSO_4 \cdot 5H_2O$, 0.4 g $FeSO_4 \cdot 7H_2O$, 3.2 g $MnCl_2 \cdot 4H_2O$ and 0.8 g $ZnSO_4 \cdot 7H_2O$ were dissolved in 500 ml of water.)

The culture broth (5 ml) was mixed with 5 ml of a mixture of chloroform and methanol (3/2) and was vigorously agitated on a mixer. It was easy to separate anthracycline-nonproducers from producers, because anthracycline producers gave orange to red chloroform extracts, whereas nonproducers did colorless ones.

Selected nonproducing strains were cultured in YS medium as described above and then were used as inocula to seed fermentation medium (30 ml medium/250 ml Erlenmeyer flask). After cultivation for 4 days, 0.3 ml of daunomycin chloride solution (1 mg/ml) was added to the culture (final cencentration 10 µg/ml) and was incubated for a further 2 days. By mixing 5 ml of the culture with 5 ml of a chloroform/methanol mixture (3/2) under vigorous agitation, the products were extracted into the chloroform layer. The chloroform extract was separated and concentrated to dryness. The residue was dissolved in 2 ml of 0.1 N HCl and then hydrolyzed at 85°C for 30 minutes to give aglycones (anthracyclinones). The aglycones were transferred into 0.1 ml of toluene under shaking. The microlitters of the toluene extract was spotted on a silica gel thin layer plate. For comparison, authentic samples of adriamycinone, daunomycinone and dihydrodaunomycinone were spotted on the same plate. The silica gel thin layer chromatographic layer was developed in a solvent system of benzene/acetone/formic acid (100/30/1). By visually comparing the amounts of adriamycinonc on silica gel thin layer plates, strains having a good efficiency of adriamycin production were selected.

Using the above-described method, those skilled in the art can easily obtain mutant strains suitable for

the present invention which have an ability to biochemically convert an anthracycline compound presented by formula (I) to adriamycin presented by formula (II).

Typical mutants capable of such conversion which were fostered by the above-explained method include mutant strains obtained from adriamycin-producing streptomycetes by mutagen treatment. One of such favorable strains is mutant 2N-267 (ATCC 31847) that was fostered by the present authors.

This mutant (2N-267) and the parent strain (Streptomyces peucetius subsp. caesius ATCC 27952) are morphologically and culturally identical, except that the former produces no anthracycline antibiotic, and no soluble pigments in various agar media presumably because of some mutation in the biosynthesis of anthracycline.

Microbiological Properties of Streptomyces Peucetius subsp. Caesius 2N-267

1. Morphology

Under a light microscope, one observes hooked aerial mycelia or incomplete spirals developed from well branched substrate mycelium. No whorl is seen. Spherical spores are 1.8 x 3.0 μ in size with smooth surface, very rarely with projections, occurring in chains.

2. Cultural properties on various media

| Medium | Growth | Color of aerial mycelium | Color of substrate mycelium | Soluble pigment |
|---|---|---|---|---|
| Sucrose nitrate agar | Moderate | No aerial mycelium forms. Occasionally poor white aerial mycelium is seen. | Pale pinkish white | None |
| Glucose asparagine agar | Poor | Occasionally poor aerial mycelium tinted in pale pinkish white is seen. | Dark | None |
| Glycerol asparagine agar | Moderate | Occasionally poor white aerial mycelium forms. | Colorless to pale yellow | None |
| Starch agar | Poor | Very rarely aerial mycelium forms. | Colorless to yellowish white tinted slightly in pink | None |
| Tyrosine agar | Good | Usually none, but occasionally poor white aerial mycelium forms. | Dark | None |
| Nutrient agar | Moderate | No aerial mycelium forms. | Pale yellowish white to yellow tinted in pink | Slightly reddish |

| Medium | Growth | Color of aerial mycelium | Color of substrate mycelium | Soluble pigment |
|---|---|---|---|---|
| Yeast extract malt extract agar | Good | No aerial mycelium forms. | Pale yellowish white to pale yellow | None |

3. Physiological properties

(1) Growth temperature range — 20 - 40°C, 28°C optimum

(2) Liquefaction of gelatin (glucose peptone gelatin) — positive

(3) Hydrolysis of starch (starch agar) — slightly positive

(4) Coagulation and/or peptonization of skim milk — none

(5) Formation of melanoid pigments (tyrosine agar and peptone yeast extract iron agar) — none

4. Utilization of various carbon sources (Pridham-Gottlib agar)

| | |
|---|---|
| L-Arabinose | - |
| D-Xylose | + |
| D-Glucose | + |
| D-Fructose | + |
| Sucrose | + |
| Inositol | - |
| L-Rhamnose | - |
| Raffinose | + |
| D-Mannitol | + |

( + indicates good growth and - no growth.)

The present authors has designated this mutant Streptomyces peucetius subsp. caesius 2N-267, and, according to the Budapest Treaty, has been placed on permanent deposit with the culture collection of the American Type Culture Collection on March 18, 1981 (accession number ATCC 31847).

Cultures or mycelia of the strains employed in the present invention are obtained by aerobically cultivating in nutrient media, and more advantageously are collected by means such as centrifugation at a stage later than the logarithmic phase of growth. In practice, it is most preferable to collect cells in the stationary phase of growth, because they are superior in the cell

yield and in the ability to convert said anthracycline antibiotics to adriamycin.

For cell production, various nutrient sources commonly employed for cultivation of streptomycetes such as carbohydrates, nitrogen sources and mineral salts are usable. For example, carbohydrates such as glucose, glycerol, maltose, sucrose, molasses, dextrin and starch; other carbon sources such as fats and oils (soybean oil, peanut oil, lard, etc.); nitrogen sources such as peptone, meat extract. soybean meal, cotton seed meal, dry yeast, corn steep liquor, yeast extract, skim milk, casein, sodium nitrate, ammonium nitrate and ammonium sulfate; mineral salts such as dipottasium phosphate, sodium chloride, calcium carbonate and magnesium sulfate; and if necessary, trace elements such as cobalt and manganese are favorably added to media.

Nutrient media are usually sterilized before inoculation. It is advisable to adjust the pH of media to 4 - 9, preferably to 6 - 8, before or after sterilization.

The cultivation is advantageously conducted in aerobic conditions usually under agitaion with or without forced aeration. Although the microorganisms can be grown in liquid media under standing culture, shaking culture and submerged culture, it is most preferred to carry out the submerged fermentation in the present invention.

Mecelia produced as described above are separated from broth filtrates by conventional methods such as sentrifugation and filtration and then washed and suspended in appropriate buffer solutions of pH 6 - 9 which do not substantially damage the conversion activity of mycelia, for example, 0.1 M Tris-HCl, pH 8.5.

In a modified conversion method of the present invention, washed mycelia harvested as described above are favorably treated by conventional methods such as enzyme digestion and sonication to an extent in which the surface structure of cells are not affected.

For mycelium digestion, enzymes derived from animal and microbial cells such as lysozyme, muramidase and Lytic Enzyme (registered trade mark; Kyowa Hakko Co., Ltd.) are usatle.

Processing conditions for enzymatic digestion of mycelia may be chosen from a wide range depending on the properties of mycelia and enzyme and the enzyme activity. In practice, 0.01 - 1 mg/ml (final concentration) of the enzyme is added to a suspension of 10 - 30 g (wet weight)/100 ml of the mycelia in a suitable buffer and is allowed to stand at a temperature of 20 - 37$^{o}$C for a period of 1 - 2 hours. The processed mycelia are collected by conventional methods such as centrifugation and are used for conversion of anthracycline antibiotics to adriamycin.

In conversion reactions of anthracycline antibiotics presented by formula (I) to adriamycin presented by formula (II), the reaction conditions such as the concentration of substrate (anthracycline antibiotics), temperature, period and pH may vary in a wide range depending on the properties and amount of the mycelia or their processed matters employed. It is easy for those skilled in the art to determine optimal reaction conditions for the particular mycelia or their processed matters by running a small-scale experiment.

It is usually advisable to execute the conversion reaction according to the present invention by vigorously agitating 10 - 100 µg/ml (final concentration) of an anthracycline antibiotic presented by formula (I) in a suspension of mycelia or their processed matters in a buffer solution of pH 6 - 9 at a temperature of 20 - 37°C for a period of 15 - 48 hours. As responsible enzyme reactions are assumed to require oxygen, the appropriate aeration improves the conversion yield and shortens the period of reaction.

As the conversion activity of mycelia or their processed matters in the present invention is stabilized by the presence of magnesium ions, it is advantageous to add magnesium chloride or magnesium sulfate to reaction mixtures. The optimal concentration of magnesium ions is usually in the range of 1- 10 mM.

Buffer solutions of pH 6 - 9 for the present invention may have a concentration in the range commonly used in most enzymes, but advantageously contain no phosphate ion.

After conversion reaction, the reaction mixture is centrifuged to give mycelia and a supernatant. As adriamycin produced by the conversion reaction is present mostly in mycelia, it is extracted from the mycelia with acetone, methanol, n-butanol or the like. Adriamycin in the supernatant solution, on the other hand, is extracted with chloroform, ethyl acetate, methyl acetate, toluene or other organic solvents. The solvent extracts recovered from the mycelia and the supernatant were combined and then concentrated to dryness to give a reddish brown preparation of crude products. Adriamycin is isolated and purified by a suitable combination of conventional methods such as column chromatography on silica gel, alumina gel, Sephadex LH-20 (Pharmacia Fine Chemicals, A.B., Sweden), weakly acidic or weakly basic ion exchange resins, ion exchange celluloses and the like; preparative thin layer chromatography on silica gel; high performance liquid chromatography and counter current distribution.

The identity with the authentic sample of adriamycin of the product obtained by the present invention was confirmed by ultraviolet and visible spectroscopy, infrared spectroscopy, silica gel thin layer chromatography of the aglycone in several solvent systems (aglycone was obtained by hydrolysis in 0.1 N HCl at 80°C for 30 minutes; silica gel 60 $F_{254}$; E. Merck, Darmstadt) and qualitative sugar analysis of the acid hydrolysate (daunosamine was detected in the acid hydrolysate by the method described in J. Antibiotics 32, 801-819 (1979)).

The below-described physicochemical properties and instrumental date of the product obtained by the present invention agreed well with those in the literature.

(1) Appearance red powder

(2) Elemental analysis

| | C | H | N |
|---|---|---|---|
| Found (%) | 59.51 | 5.40 | 2.65 |
| Calculated for $C_{27}H_{29}NO_{11}$ | 59.66 | 5.38 | 2.57 |

(3) Molecular weight 543.5

(4) Melting point 199 - 201°C (decomposition) (for HCl salt)

(5) Ultraviolet and visible spectroscopy

| Solvent | $\lambda_{max}$ nm($E^{1\%}_{1\,cm}$) |
|---|---|
| 90 % MeOH | 496(217) 529(118)<br>479(219) 233(673) |
| 0.1 N HCl-90 % MeOH | 496(203) 530(113)<br>476(207) 233(587) |
| 0.1 N NaOH-90 % MeOH | 595(262) 562(276)<br>290(s)(190) 252(661) |

(6) Infrared spectroscopy (KBr tablet)

$\nu^{max}_{cm^{-1}}$ : 3350, 2950, 1720, 1620, 1580, 1530, 1420 1280, 1230, 1210, 1110, 1080, 980, 910, 870

(7) PMR spectroscopy (100 MHz; chloroform)

| | | |
|---|---|---|
| H-1 (8.00) | H-2 (7.75) | H-3 (7.35) |
| H-1' (5.50) | H-7 (5.25) | H-3',H-5' (4.15) |
| $\underline{OCH_3}$ (4.05) | H-4' (3.45) | $CO\underline{CH_2}OH$ (3.30) |
| H-10 (3.05) | H-8 (2.20) | $CH_2$-2' (1.70) |
| $\underline{CH_3}$-5' (1.35) | | |

The physicochemical properties and instrumental data of adriamycin are reported in the following references:

Reference 1) Tetrahedron Letters 13, 1007 (1969)

2) Biotechnol. Bioeng. 11, 1101 (1969)

3) Recent Results in Cancer Res. p.9, Berlin-Heidelberg-New York, Springer Verlag, 1972

The examples which follow are to illustrate the present invention in detail.

EXAMPLE 1

| | |
|---|---|
| Potato starch | 1.5 % |
| Glucose | 1.0 % |
| Soybean meal (ESUSAN MEAT; Ajinomoto Co.) | 1.0 % |
| Yeast extract | 0.1 % |
| Dipotassium phosphate | 0.1 % |
| Magnesium sulfate | 0.1 % |
| Sodium chloride | 0.3 % |
| Mineral solution* | 0.125 % |

pH 7.4

(* 2.8 g of $CuSO_4 \cdot 5H_2O$, 0.4 g of $FeSO_4 \cdot 7H_2O$, 3.2 g of $MnCl_2 \cdot 4H_2O$ and 0.8 g of $ZnSO_4 \cdot 7H_2O$ were dissolved in 500 ml of water to make the mineral solution.)

Unless specified otherwise, the percentage in the present invention is expressed in the weight/volume base.

The seed culture was prepared by inoculating a loopful of spores of Streptomyces peucetius subsp. caesius 2N-267 from the slant culture into a 500 ml Erlenmeyer flask containing 50 ml of the sterile medium specified above, and shake-culturing the flask for 48 hours at 28°C on a rotary shaker (230 rpm).

| | |
|---|---|
| Sucrose | 5.0 % |
| Soybean meal (ESSUSAN MEAT) (see above) | 2.0 % |
| Corn steep liquor | 0.2 % |
| Yeast extract | 0.2 % |
| Dipotassium phosphate | 0.1 % |
| Magnesium sulfate | 0.05 % |
| Calcium carbonate | 0.2 % |
| Mineral solution (see above) | 0.2 % |

pH 7.4

The above-specified medium (5 liters) were prepared and distributed in 50 ml volumes into 500 ml Erlenmeyer flasks. After sterilization, 1 ml each of the above-described seed culture was inoculated to the flasks and incubated at 28°C for 84 hours on a rotary shaker (230 rpm).

The mycelia were collected from the broth by centrifugation; washed with a suitable volume of 0.1 M Tris-HCl

buffer, pH 8.5; and suspended in 5 liters of the buffer. Fifty milliliters each of the mycelium suspension was distributed in 500 ml Erlenmeyer flasks. One milliliter each of 0.5 mg/ml of daunomycin hydrochloride solution was added to the flasks (total amount of daunomycin = 50 mg) and the flasks were incubated at 28°C for 20 hours on a rotary shaker. After reaction, the mycelia were separated from the supernatant solution by centrifugation. Adriamycin in the supernatant was extracted twice with 2 liters each of chloroform, while the product in the mycelia was done twice with 2 liters each of acetone. The acetone extracts were combined and concentrated to a small volume under reduced pressure. Adriamycin was extracted twice from the concentrate with 1 liter each of chloroform. The chloroform extracts from the mycelia and from the supernatant were combined and concentrated under reduced pressure to give tar.

The above-described crude tarry matter was dissolved in a small volume of chloroform; applied in a linear fashion 1.5 cm from the bottom edge on preparative silica gel thin layer plates (Silica gel $PF_{254}$; E. Merck, Darmstadt) and developed in a solvent system of chloroform/methanol/water/acetic acid (75/25/5/5). The band of adriamycin at Rf 0.2 was satisfactorily separated from unchanged daunomycin (substrate) at Rf 0.33 and from dihydrodaunomycin (a metabolite of daunomycin) at

Rf 0.25. The area of adriamycin was scraped off from the thin layer plates. Adriamycin was extracted from the silica gel with a mixture of chloroform and methanol (4/1; v/v) and then concentrated to dryness in vacuo. For purification, the red residue was dissolved in 50 ml of 0.1 M acetate (pH 3.0) and washed twice with 20 ml each of toluene. After adjusting to pH 6.8 with 4 N NaOH under cooling in ice water, the aqueous layer was subjected to extraction with 200 ml in total of chloroform. The chloroform extract was washed with water, dried over anhydrous sodium sulfate and then concentrated to about 3 ml under reduced pressure. Adriamycin in the concentrate was forced to precipitate by addition of 20 ml of n-hexane and was recovered by filtration. Drying in vacuo gave 12.6 mg of red powder of adriamycin. The yield of conversion was calculated to be 25 %.

EXAMPLE 2

According to the same method as explained in Example 1, mycelia of Streptomyces peucetius subsp. caesius 2N-267 were prepared from 5 liters of culture broth. Following rinsing with 1 liter of 0.1 M Tris-HCl buffer, pH 8.5, the mycelia were suspended in 5 liters of the same buffer containing 0.1 mg/ml (final concentration) of Lytic Enzyme #2 (Kyowa Hakko Co., Ltd.) and were allowed to stand at 37°C for one hour. After enzyme

treatment, the mycelia were recovered by centrifugation at 4°C and washed with 1 liter of 5 mM magnesium chloride. The processed mycelia were resuspended in 5 liters of 0.1 M Tris-HCl, pH 8.5, containing 5 mM magnesium chloride and then were mixed with 50 ml of 1 mg/ml of daunomycin hydrochloride. Fifty milliliters each of the suspension was distributed in 500 ml Erlenmeyer flasks and was kept shaken for 20 hours on a rotary shaker (see above).

The processed mycelia and the supernatant solution were separated by centrifugation. The supernatant was subjected to extraction with 1.5 liters of chloroform. The processed mycelia were treated twice with 2 liters each of acetone. The acetone extracts were united and concentrated to a small volume under reduced pressure. Adriamycin was extracted from the acetone concentrate with 1.5 liters of chloroform. The chloroform extracts derived from the supernatant and the mycelia were combined and concentrated to dryness to provide a crude mixture of products. Using the same thin layer chromatographic method as described in Example 1, adriamycin was purified. Final purification consisting of dissolving in acidic water followed by back-extraction into an organic solvent at neutral pH gave 19.2 mg of pure adriamycin. The percentage of conversion was found to be 38.4 %.

EXAMPLE 3

By the same procedures as explained in Examples 1 and 2, the suspensions of the intact mycelia and the Lytic Enzyme #2-treated mycelia of Streptomyces peucetius subsp. caesius 2N-267 were prepared from 5 liters each of the culture broth. After 50 ml each of 1 mg/ml of dihydrodaunomycin hydrochloride in water was mixed well with the mycelium suspensions, the reaction mixtures were distributed in 50 ml portions into 500 ml Erlenmeyer flasks and were shaken for 20 hours on a rotary shaker. The same isolation and purification method as detailed in Example 1 provided adriamycin. The yield of adriamycin with the enzyme-nontreated mycelia was 11.9 mg (conversion percentage 23.9 %), whereas that with the enzyme-treated mycelia was 17.7 mg (conversion percentage 35.4 %).

EXAMPLE 4

The mycelium suspension of Streptomyces peucetius subsp. caesius 2N-267 was produced from 5 liters of culture broth by the same method as shown in Example 1. To the mycelium suspension, 50 ml of 1 mg/ml of deoxodaunomycin hydrochloride in water was added and mixed well. The mixture was distributed in 50 ml portions into 500 ml Erlenmeyer flasks and kept stirred for 20 hours on a rotary shaker. The same procedure for isolation and purification as described in Example 1 yielded 10.8 mg of adriamycin (conversion percentage 21.6 %).

EXAMPLE 5

By utilizing the same method as explained in Example 1, 5 liters each of the culture broth of *Streptomyces peucetius* subsp. *caesius* 2N-267 gave the mycelium suspensions. After 50 ml of 1 mg/ml of carminomycin hydrochloride or dihydrocarminomycin hydrochloride was added and stirred well, 50 ml each of the suspension was distributed in 500 ml Erlenmeyer flasks and then shaken for 20 hours on a rotary shaker.

Adriamycin was extracted and purified by the same process as explained in Example 1. The yield of adriamycin was 14.6 mg from carminomycin (conversion percentage 29.2 %) and 12.8 mg from dihydrocarminomycin (conversion percentage 25.6 %).

EXAMPLE 6

The seed cultures of *Streptomyces peucetius* subsp. *caesius* ATCC 27952 and *Streptomyces peucetius* subsp. *carneus* ATCC 21354 were prepared by the same method as described in Example 1. One milliliter each of the seed cultures was inoculated to 500 ml Erlenmeyer flasks containing 50 ml of the fermentation medium specified in Example 1, and was cultivated at 28°C for 90 hours

on a rotary shaker (see above). The mycelia of the two streptomycetes were collected from one flask each of the culture broths by centrifugation and washed with 50 ml of 0.1 M Tris-HCl buffer. The mycelia were suspended in 50 ml of the same buffer containing 5 mM magnesium chloride and poured in a 500 ml Erlenmeyer flask. One milliliter of 0.5 mg/ml of daunomycin chloride was added to each flask (final concentration 10 µg/ml) and the flasks were kept stirred at 28°C for 20 hours on a rotary shaker (see above). Five milliliter each of the suspensions was collected from the flasks and was mixed with 2 ml of methanol and then with 3 ml of chloroform on a mixer. The chloroform layer containing anthracycline compounds was separated and concentrated to dryness *in vacuo*. The residue was dissolved in 3 ml of 0.1 N HCl and hydrolyzed at 85°C for 30 minutes, giving adriamycinone, daunomycinone and dihydrodaunomycinone corresponding to adriamycin, daunomycin and dihydrodaunomycin, respectively. These aglycones were separated from the hydrolysate by extracting twice with 3 ml each of chloroform and the chloroform extracts were combined and concentrated to dryness under reduced pressure. The residue was dissolved in 0.1 ml of chloroform. Ten microliters of the solution was spotted on a silica gel thin layer plate (silica gel 60 $F_{254}$, E. Merck, Darmstadt) and developed in a solvent mixture of benzene, acetone and

formic acid (100/30/1). The Rf values of adriamycinone, daunomycinone and dihydrodaunomycinone were found to be 0.20, 0.35 and 0.16, respectively. The amount of adriamycinone in the corresponding spot was measured at 490 nm in a Shimazu thin layer chromatoscanner CS-910 and was calibrated from the standard curve of authentic adriamycinone. The concentration of adriamycin in reaction mixtures was calculated on the basis of the amount of adriamycinone. The results of thin layer chromatographic analysis are summarized in the following table. For comparison, the same conversion experiment was conducted with the above-described mutant 2N-267.

| Strains | Amount of daunomycin added (µg/ml) | Amount of adriamycin produced (µg/ml) |
|---|---|---|
| Streptomyces peucetius subsp. caesius ATCC 27952 | None<br>10 | 0.1<br>3.4 |
| Streptomyces peucetius subsp. carneus ATCC 21354 | None<br>10 | 0.05<br>0.7 |
| Streptomyces peucetius subsp. caesius 2N-267 | None<br>10 | 0<br>5.8 |

What is claimed is:

1. A process for production of adriamycin which consists of biochemically converting an anthracycline antibiotic presented by formula (I)

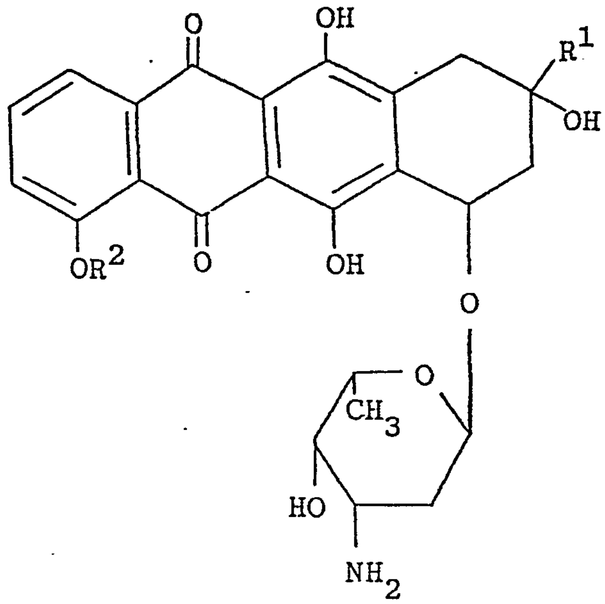

(I)

wherein

$R^1$ is acetyl, 1-hydroxyethyl or ethyl;

and $R^2$ is a hydrogen atom or methyl;

but, when $R^1$ is ethyl, $R^2$ is methyl,

to adriamycin presented by formula (II)

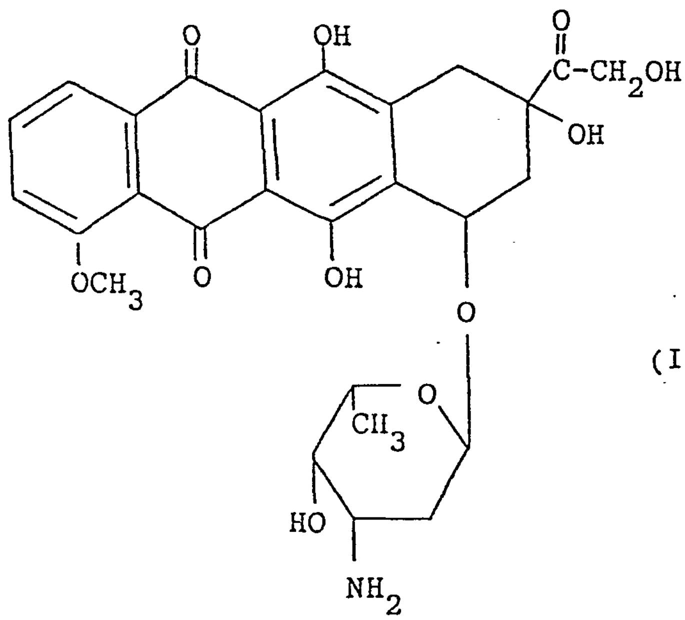

(II)

in the presence of the mycelium or its processed matters of a streptomycete having such conversion ability whereby adriamycin is isolated and purified by conventional methods.

2. The process for production of adriamycin according to claim 1 wherein the processed matter of the mycelium of a streptomycete is prepared by treatment with lytic enzyme.

3. The process for production of adriamycin according to claim 1 or claim 2 wherein the streptomycete is selected from the group of Streptomyces peucetius subsp. caesius ATCC 27952, Streptomyces peucetius subsp. carneus ATCC 21354 and their mutants.

4. The process for production of adriamycin according to claim 3 wherein the mutant of Streptomyces peucetius subsp. caesius ATCC 27952 is Streptomyces peucetius subsp. caesius 2N-267 ATCC 31847.

European Patent Office

# EUROPEAN SEARCH REPORT

0061737

Application number

EP 82 10 2532

| Category | Citation of document with indication, where appropriate, of relevant passages | Relevant to claim | CLASSIFICATION OF THE APPLICATION (Int. Cl. 3) |
|---|---|---|---|
| P,X | THE JOURNAL OF ANTIBIOTICS, vol. XXXIV, no. 9, 1981, pages 1229-1231, Published Japan antibiotics research association; T.OKI et al.: "Microbial conversion of daunomycin, carminomycin I and feudomycin A to adriamycin". *The whole document* | 1-4 | C 12 P 19/56// (C 12 F 19/56 C 12 R 1/465) C 07 H 15/24 |
| A,X | CHEMICAL ABSTRACTS, vol. 90, no. 25, 18th June 1979, page 269, no. 199993n, Columbus Ohio (USA); G.J.SHAW et al.: "Propionate precursors in the biosynthesis of daunomycin and adriamycin: a carbon-13 nuclear magnetic resonance study". & PHYTOCHEMISTRY 1979, 18(1), 178-9 *Abstract* | 1,2,3 | TECHNICAL FIELDS SEARCHED (Int. Cl. 3) |
| A | US-A-4 013 515 (FLORENT) *The whole document* | 1 | C 07 H 15/00 C 12 P 19/00 C 12 R 1/00 |
| A,X | EP-A-0 012 159 (SANRAKU) *Pages 0-3; claims; pages 1-3* | 1,3-4 | |
| A | HAMAO UMEZAWA: "Index of antibiotics from actinomycetes", vol. II, 1978, page 122, Japan scientific societies press, Toyko (JP); "Adriamycin". | 1,2 | |

The present search report has been drawn up for all claims

| Place of search | Date of completion of the search | Examiner |
|---|---|---|
| THE HAGUE | 07-07-1982 | RAJIC M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

**0061737**

Application number

# EUROPEAN SEARCH REPORT

EP 82 10 2532

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| D,A | THE JOURNAL OF ANTIBIOTICS, vol. XXXIII, no. 10, 1980, pages 1158-66, Editor: Japan Antibiot. Res. Associat.; A.YOSHIMOTO et al.: "Microbial conversion of anthracyclinones to daunomycin by blocked mutants of streptomyces coeruleorubidus" *The whole document* --- | 1,2,3 | |
| A | THE JOURNAL OF ANTIBIOTICS, vol. XXXI, no. 4, 1978, pages 336-42, Editor: Japan Antibiot. Res. Assoc.; V.P.MARSHALL et al.: "Microbial metabolism of anthracycline antibiotics daunomycin and adriamycin". *The whole document* ----- | 1,2 | TECHNICAL FIELDS SEARCHED (Int. Cl. 3) |

The present search report has been drawn up for all claims

| Place of search | Date of completion of the search | Examiner |
|---|---|---|
| THE HAGUE | 07-07-1982 | RAJIC M. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document